# EUROPEAN PATENT APPLICATION

(11) **EP 4 755 368 A1**
(43) Date of publication of application: **10.06.2026**
(21) Application number: 24852133.8
(22) Date of filing: 25.07.2024
(51) Int. Cl.: A61K 9/00, A61K 47/42, A61K 47/26, A61K 38/17, A61P 27/02

(54) **EYE DROP COMPOSITION FOR PREVENTING OR TREATING OCULAR NEOVASCULAR DISEASES, COMPRISING COLLAGEN TYPE I AND PIGMENT EPITHELIUM-DERIVED FACTOR PEPTIDE AS ACTIVE INGREDIENTS**

(30) Priority: 04.08.2023 KR 20230102223; 24.07.2024 KR 20240097809
(71) Applicant: NexThera Co., Ltd., Busan 48931 (KR)
(72) Inventor: ROH, Kug-Hwan, Busan 49418 (KR); LIM, Nayoung, Busan 47002 (KR); LEE, You Song, Busan 48435 (KR); LEE, Kyungsun, Incheon 22002 (KR)
(74) Representative: Mewburn Ellis LLP
(86) International application number: PCT/KR2024/010779
(87) International publication number: WO 2025/033777

(57) **Abstract**

The present invention relates to an eye drop composition for preventing or treating ocular neovascular diseases, comprising collagen type I and a pigment epithelium-derived factor peptide as active ingredients. The present invention provides an eye drop composition capable of most stably storing for a long period of time a pigment epithelium-derived factor peptide, which is an active ingredient exhibiting an anti-neovascular effect on ocular neovascular diseases, and as it is confirmed that the eye drop composition, according to the present invention, exhibits a therapeutic or ameliorating effect on ocular neovascular diseases even when the eye drop composition contains a low concentration of a pigment epithelium-derived factor (PEDF) peptide represented by SEQ ID NO: 1, which is an active ingredient, the present invention provides the eye drop composition exhibiting a therapeutic or ameliorating effect on ocular neovascular diseases and having excellent storage stability.

## Description

### Technical Field

The present disclosure relates to an eye drop composition for preventing or treating an ocular neovascular disease, including collagen type I and a pigment epithelium-derived factor peptide as an active ingredient.

### Background Art

The macula is the neural tissue located at the center of the retina where most photoreceptor cells are concentrated, and is responsible for central vision as an area where images of objects are formed. Macular degeneration is an ocular disease that often progresses with age in which vision impairment is caused due to degeneration of the macula, and in the early stages of the disease, vision becomes blurred and near vision becomes distorted, eventually leading to blindness.

Age-related macular degeneration (AMD) refers to a chronic, progressive degenerative condition of the macula, which causes central vision loss. AMD is currently the most common cause of blindness in people aged over 65 and the third leading cause of blindness worldwide, and approximately 30 million people worldwide suffer from the disease, with 500,000 patients losing their vision due to this disease each year. In Korea, 16.5% of people aged over 65 are found to have early-stage AMD, and the age of onset of AMD is gradually decreasing from the 60s to the 40s.

AMD is broadly classified into two types: atrophic and exudative. The atrophic type accounts for 90% of AMD cases and is a disease in which central vision gradually declines due to abnormalities in the retinal pigment epithelium in the macula, and currently, there are no treatments to restore vision beyond smoking cessation, UV protection, and antioxidant supplementation. The exudative type accounts for approximately 10% of AMD cases and causes faster and more severe visual impairment than the atrophic type. The exudative case is often accompanied by fundus findings such as choroidal neovascularization, retinal pigment epithelial detachment, sensory retinal detachment, and retinal pigment epithelial rupture, and it is known that 70-90% of blindness due to AMD is caused by exudative lesions. Choroidal neovascularization (CNV) is a disease in which new blood vessels increase in the choroid layer of the eye and is associated with AMD.

Pigment epithelium-derived factor (PEDF) is a protein with anti-angiogenic effects, which is considered a material for treating various diseases caused by angiogenesis, and currently, research is being conducted to use PEDF as a treatment for macular degeneration, but it has been pointed out that the short half-life requires repeated injections and the concentration range that can be applied for disease treatment is narrow. Therefore, research is needed to solve the problems of PEDF and maximize its effectiveness in treating macular degeneration.

### [Prior-Art Documents]

### [Patent Documents]

Korean Patent Application Publication No. 10-2012-0130752 (published on December 3, 2012)

Korean Patent Application Publication No. 10-2018-0034518 (published on April 4, 2018)

### Disclosure of the Invention

### Technical Goals

An object of the present disclosure is to confirm that an eye drop composition including collagen type I and a pigment epithelium-derived factor (PEDF) peptide has an antiangiogenic effect on ocular neovascular disease, to suggest suitable eye drop conditions for most safely storing PEDF peptide as an active ingredient in the eye drop, and to provide the most suitable content range of collagen type I and PEDF peptide that exhibits a therapeutic or improvement effect on ocular neovascular disease when instilled into the eye.

### Technical Solutions

The present disclosure provides an eye drop composition for preventing or treating an ocular neovascular disease, including collagen type I and a PEDF peptide. Advantageous Effects

According to the present disclosure, an eye drop composition is provided that can most safely store PEDF peptide, which is an active ingredient showing an antiangiogenic effect on ocular neovascular disease, for a long period of time, and by confirming that the eye drop composition according to the present disclosure shows a therapeutic or improvement effect on ocular neovascular disease even when it contains a low concentration of the PEDF peptide represented by SEQ ID NO: 1, which is an active ingredient, it is possible to provide an eye drop composition that shows a therapeutic or improvement effect on ocular neovascular disease and has excellent storage stability.

### Brief Description of Drawings

FIG. 1 shows results of evaluating a therapeutic or improvement effect on CNV of an eye drop composition under 1x A5S buffer conditions prepared according to Preparation Example 1 of the present disclosure.
FIG. 2 shows results of evaluating a therapeutic or improvement effect on choroidal neovascularization of an eye drop composition under 1x A55S buffer conditions prepared according to Preparation Example 2 of the present disclosure.
FIG. 3 shows results of evaluating a therapeutic or improvement effect on CNV of an eye drop composition under 0.1x PBS buffer conditions prepared according to Preparation Example 3 of the present disclosure.
FIG. 4A shows results of evaluating a content range of a PEDF peptide that exhibits a therapeutic or improvement effect on CNV in an eye drop composition under 1x A55S buffer conditions prepared according to Preparation Example 2 of the present disclosure.
FIG. 4B shows results of evaluating a maximum content range of a PEDF peptide that exhibits a therapeutic or improvement effect on CNV in an eye drop composition under 1x A55S buffer conditions prepared according to Preparation Example 2 of the present disclosure.
FIG. 5 shows results of evaluating a therapeutic or improvement effect on CNV using three types of collagen type I (TheraCol (Porcine), Pepsin Soluble Collagen (Bovine), VitroCol (Human)) from different manufacturers and biological sources in an eye drop composition under 1x A55S buffer conditions prepared according to Preparation Example 4 of the present disclosure.
FIG. 6 shows results of evaluating a therapeutic or improvement effect on CNV using two types of collagen type I (Matrixen^{™}-PSP (Porcine), Pepsin Soluble Collagen (Bovine)) from different manufacturers and biological sources in an eye drop composition under 1x AS buffer conditions of pH 5.0, 5.5, and 6.0 prepared according to Preparation Example 5 of the present disclosure.
FIG. 7 shows results of evaluating a therapeutic or improvement effect on CNV according to pH in an eye drop composition under 1x AS buffer conditions of pH 4.0, 4.2, 4.5, 4.7, 5.0, or 5.3 prepared according to Preparation Example 6 of the present disclosure.
FIG. 8 shows results of evaluating a therapeutic or improvement effect on CNV by adding 0.005% or 0.01% of benzalkonium chloride (BAK) or 0.001% of polyquaternium-1 (PQ1) to an eye drop composition in which a preservative is added to 1x AS buffer conditions prepared according to Preparation Example 7 of the present disclosure.
FIG. 9 shows results of evaluating a maximum content range of a PEDF peptide that exhibits a therapeutic or improvement effect on CNV in a composition in which 0.01% BAK is added to 1x A5S buffer conditions prepared according to Preparation Example 8 of the present disclosure.
FIG. 10 shows results of evaluating a therapeutic or improvement effect on CNV using four PEDF peptides with different manufacturing numbers manufactured by PolyPeptide Laboratories (manufacturing numbers FSC04053, FSC04084, AW19111, or 23-22-0147-01) in a composition in which 0.01% BAK is added to 1x A5S buffer conditions prepared according to Preparation Example 9 of the present disclosure.
FIG. 11A shows results of evaluating a maximum content range of collagen type I (Pepsin Soluble Collagen, Bovine) that exhibits a therapeutic or improvement effect on CNV in an eye drop composition under 1x A5S buffer conditions prepared according to Preparation Example 10 of the present disclosure.
FIG. 11B shows results of evaluating a minimum content range of collagen type I (Pepsin Soluble Collagen, Bovine) that exhibits a therapeutic or improvement effect on CNV in a composition in which 0.01% BAK is added to 1x A5S buffer conditions prepared according to Preparation Example 11 of the present disclosure.

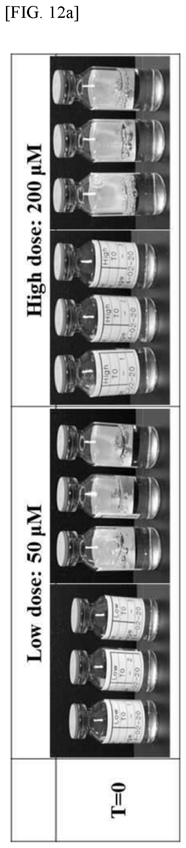

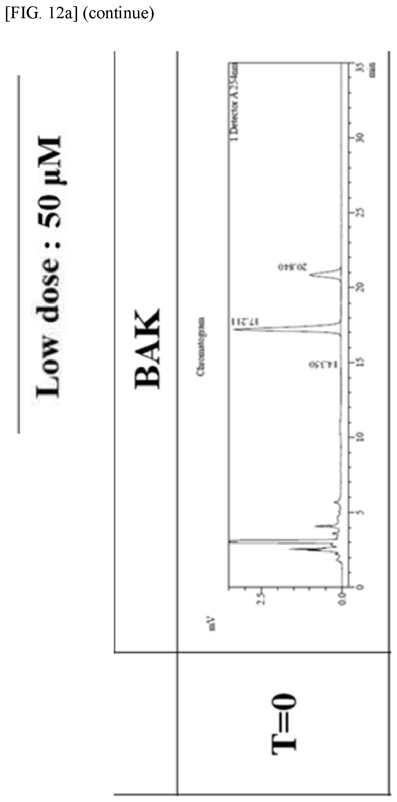

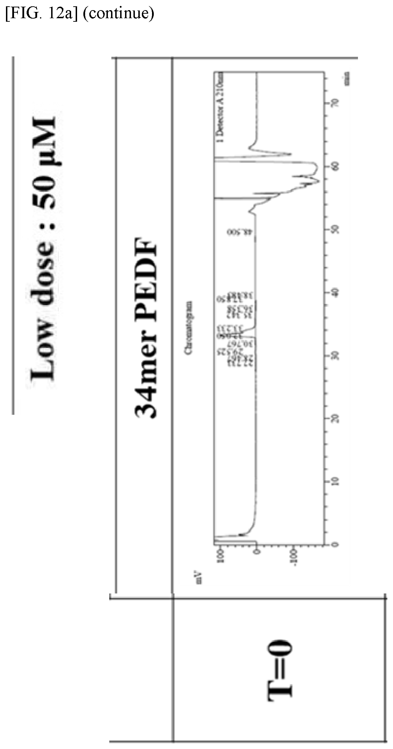

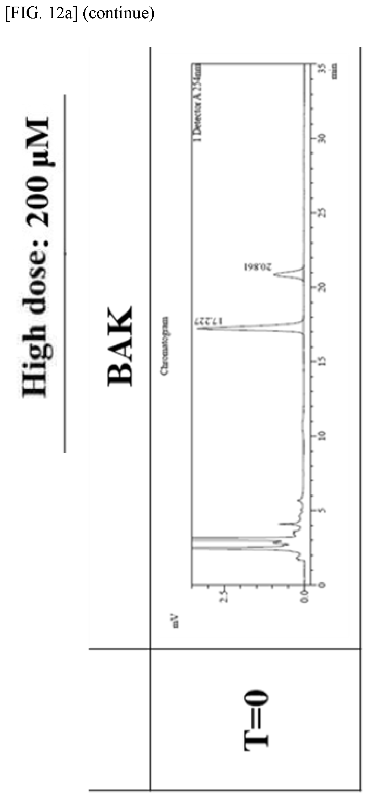

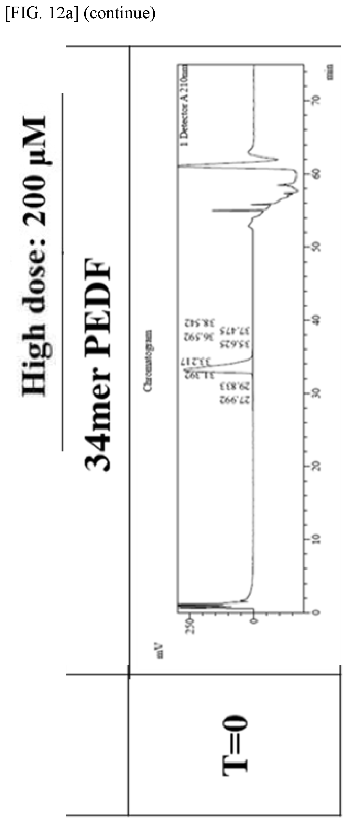

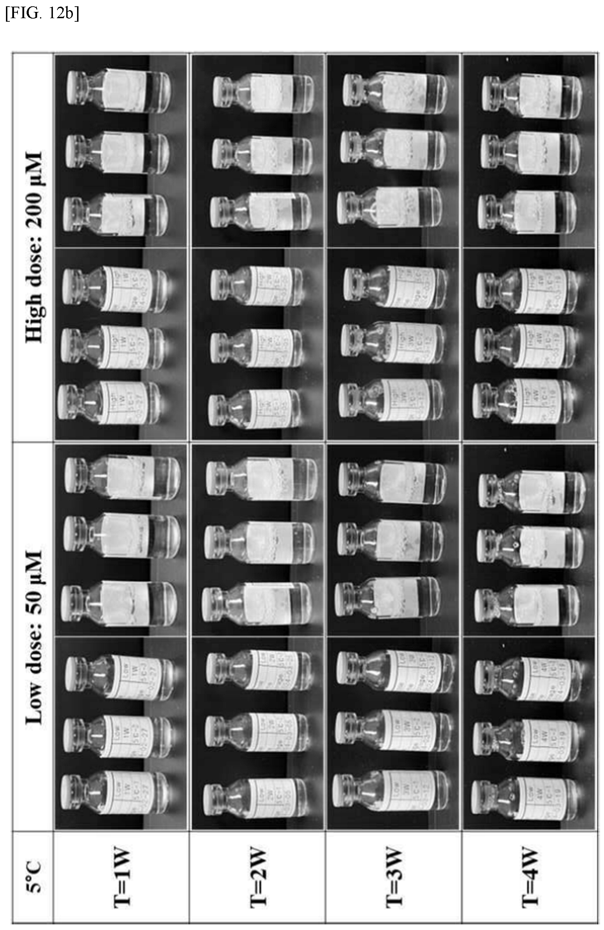

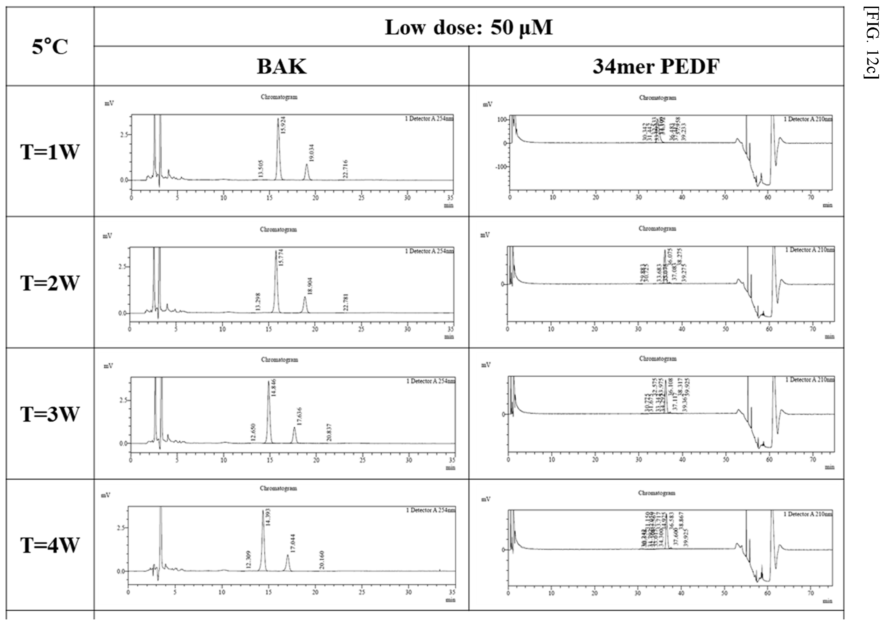

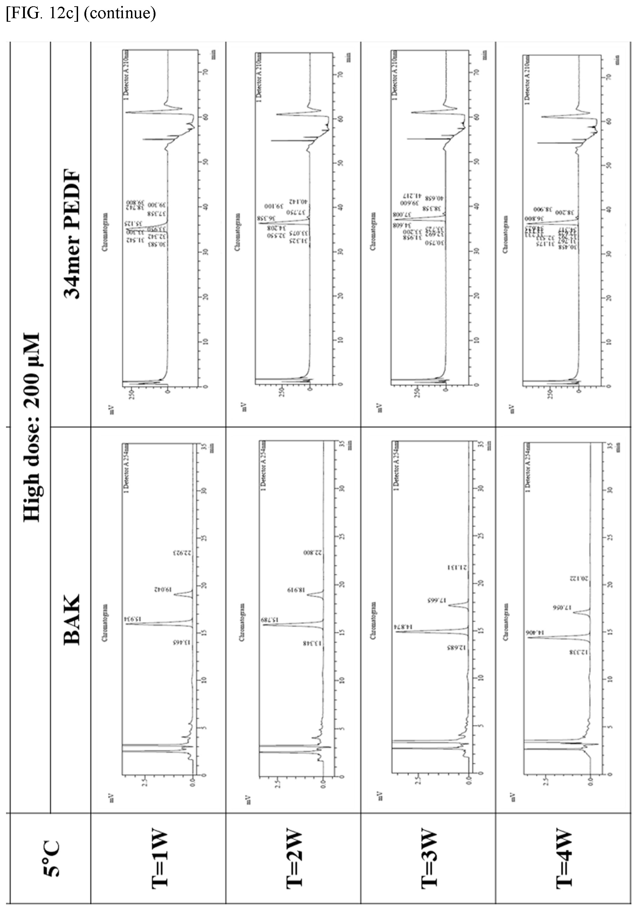

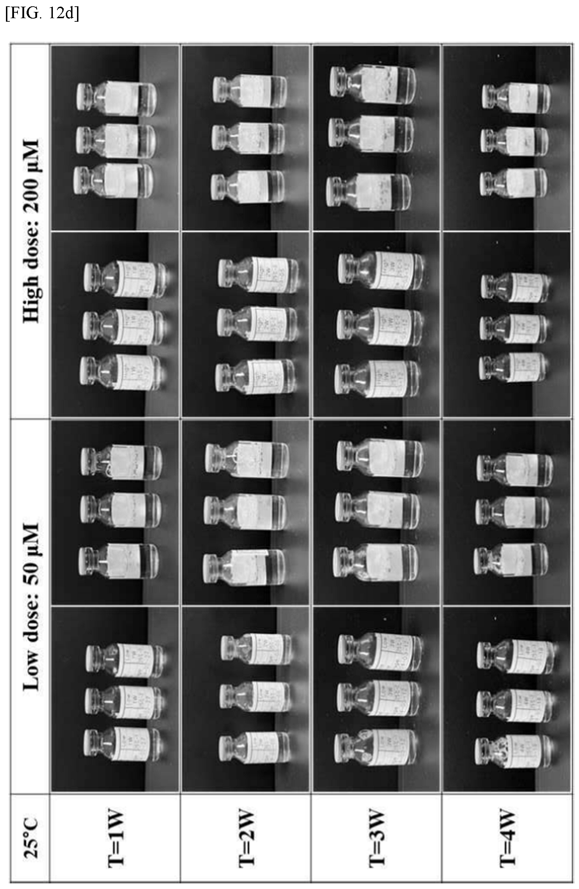

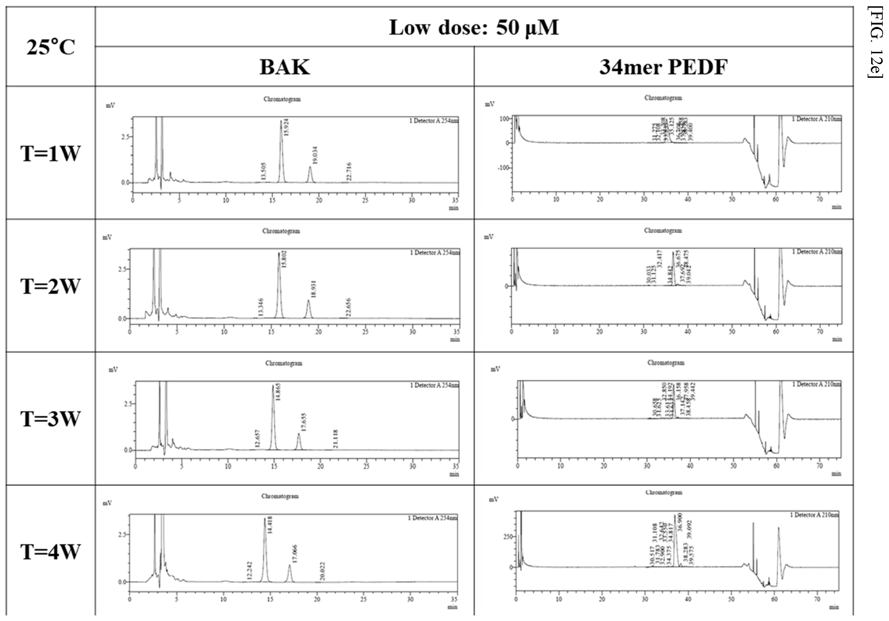

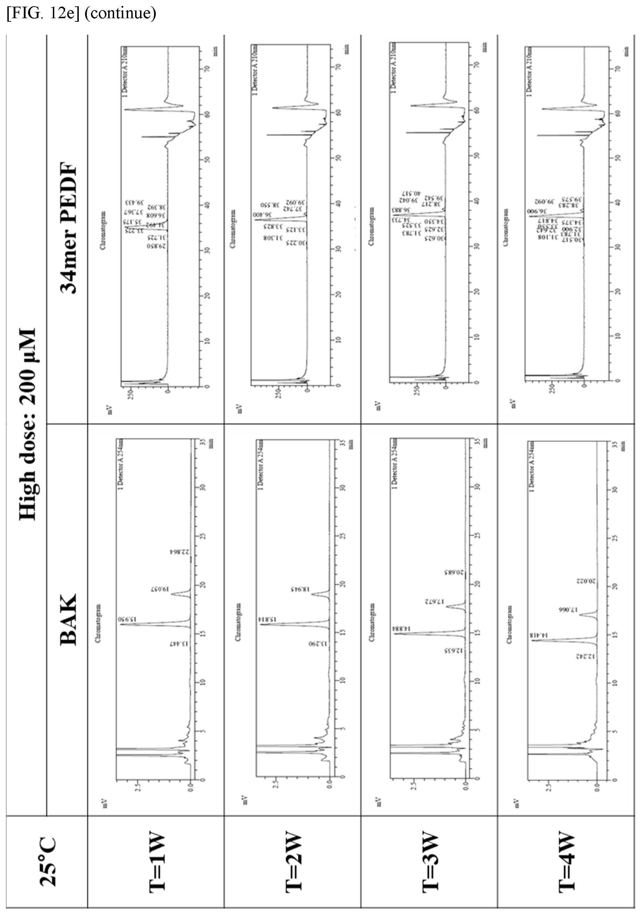

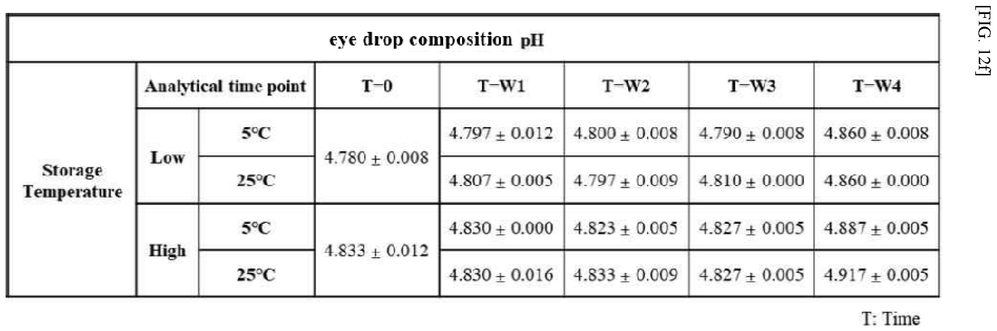

FIG. 12 shows results of storing final eye drop compositions prepared according to Preparation Example 12 of the present disclosure under refrigerated conditions (5±3°C) or room temperature conditions (25±2°C) for 4 weeks, checking for color, transparency, and formation of precipitates through weekly visual inspection, measuring pH, and analyzing contents of preservative BAK and PEDF peptide contained in the eye drop compositions using reverse phase ultra-high performance liquid chromatography (RP-UPLC). FIG. 12A shows results of analysis of transparency, BAK, and PEDF content at week 0 of storage of a final eye drop composition of the present disclosure prepared according to Preparation Example 12. FIG. 12B shows results of evaluating color, transparency, and formation of precipitates through weekly visual inspection after storing a final eye drop composition of the present disclosure prepared according to Preparation Example 12 under refrigerated conditions (5±3°C) for 4 weeks. FIG. 12C shows results of evaluating changes in BAK and PEDF contents after storing a final eye drop composition of the present disclosure prepared according to Preparation Example 12 under refrigerated conditions (5±3°C) for 4 weeks. FIG. 12D shows results of evaluating color, transparency, and formation of precipitates through weekly visual inspection after storing a final eye drop composition of the present disclosure prepared according to Preparation Example 12 under room temperature conditions (25±2°C) for 4 weeks. FIG. 12E shows results of evaluating changes in BAK and PEDF contents after storing a final eye drop composition of the present disclosure prepared according to Preparation Example 12 under room temperature conditions (25±2°C) for 4 weeks. FIG. 12F is a table showing results of storing final eye drop compositions prepared according to Preparation Example 12 of the present disclosure under refrigerated conditions (5±3°C) or room temperature conditions (25±2°C) for 4 weeks, checking for color, transparency, and formation of precipitates through weekly visual inspection, measuring pH, and analyzing changes in contents of preservative BAK and PEDF peptide contained in the eye drop compositions.

### Best Mode for Carrying Out the Invention

The terms used in the present specification have been selected as general terms widely used at present as possible while considering the functions of in the present disclosure, but may vary depending on the intention of those skilled in the art, precedents, the emergence of new technologies, and the like. In addition, in certain cases, there are terms arbitrarily selected by the applicant, and in this case, their meanings will be described in detail in the relevant detailed description. Therefore, the term used in the present disclosure should be defined based on the meaning of the term and the overall content of the present disclosure, rather than simply the name of the term.

Unless otherwise defined herein, all terms used herein including technical or scientific terms have the same meanings as those generally understood by one of ordinary skill in the art. Terms defined in dictionaries generally used should be construed to have meanings matching contextual meanings in the related art and are not to be construed as an ideal or excessively formal meaning unless otherwise defined herein.

Numerical ranges are inclusive of the values defined in the ranges above. Every maximum numerical limitation given throughout the present specification includes every lower numerical limitation, as if that lower numerical limitation were expressly written out. Every minimum numerical limitation given throughout the present specification includes every higher numerical limitation, as if that higher numerical limitation were expressly written out. Every numerical limitation given throughout the present specification will include every better numerical range within the broader numerical range, as if that narrower numerical limitation were expressly written out.

Hereinafter, the present disclosure will be described in more detail.

The present disclosure provides an eye drop composition for preventing or treating an ocular neovascular disease, including collagen type I and a pigment epithelium-derived factor (PEDF) peptide.

The PEDF peptide consists of a 34 amino acid sequence represented by SEQ ID NO: 1 (DPFFK VPVNK LAAAV SNFGY DLYRV RSSTS PTTN), and may be included in the eye drop composition in a therapeutically effective amount that exhibits an effect of inhibiting ocular neovascularization.

The therapeutically effective amount may vary depending on the subject to an appropriate effective dose, and if the subject is a human, it may vary depending on factors such as age, weight, height, sex, general medical condition, and previous medical history. The subject may be a mammal including a human, and mammals may include a human, a mouse, a rat, a dog, and a cat.

In a specific embodiment of the present disclosure, when the subject is a mouse, the eye drop composition of the present disclosure may include the PEDF peptide in an amount of 0.188 ng/10 µL (0.005 µM) to 37.6 µg/10 µL (1000 µM), and specifically, the concentration exhibiting CNV efficacy may be included in an amount of 7.52 ng/10 µL to 37.6 µg/10 µL. The eye drop composition of the present disclosure may be used by instilling the PEDF peptide in a range of 7.52 ng to 37.6 µg into the eye once or several times a day at a dosage of 10 µL, and preferably, may be used by local instillation into the eye at a dosage range in which the effective amount of the PEDF peptide is0.376 µg/kg/day (7.52 ng/eye/day) to 1.88 mg/kg/day (37.6 µg/eye/day).

In addition, in another specific embodiment of the present disclosure, when the subject is a human, the eye drop composition of the present disclosure may include the PEDF peptide in an amount of 0.94 ng/50 µL to 94 mg/50 µL, and specifically, the concentration exhibiting CNV efficacy may be included in an amount of 37.6 ng/50 µL to 94 mg/50 µL. The eye drop composition of the present disclosure may be used by instilling the PEDF peptide in a range of 37.6 ng to 94 mg into the eye once a day at a dosage of 50 µL to 100 µL, and preferably, may be used by local instillation into the eye at a dosage range in which the effective amount of the PEDF peptide is 0.63 ng/kg/day (37.6 ng/eye/day) to 1.57 mg/kg/day (94 mg/eye/day).

The eye drop composition may include a buffer that is harmless to the human body so as to safely store the active ingredient, and the buffer may include sodium acetate, sorbitol, and polysorbate 80, and may include BAK or PQ-1 as a preservative. More specifically, the eye drop composition may include a buffer including 9.9 mM to 10.1 mM sodium acetate, 4.9 wt% to 5.1 wt% sorbitol, and 0.009 wt% to 0.011 wt% polysorbate 80 in order to safely store the active ingredient under various temperature conditions for a long period of time, and may include 0.005% or 0.01% BAK or 0.001% PQ-1 as a preservative. The pH of the buffer may be adjusted by adding acetic acid, and the A5S buffer may form a pH of 4.9 to 5.1, and the A55S buffer may form a pH of 5.4 to 5.6. Since the eye drop composition includes the buffer, it may be stored for 4 weeks at a temperature ranging from 1°C to 30°C.

The eye drop composition may exhibit a pharmacological effect by instilling the PEDF peptide into the eye once a day at a dosage ranging from 37.6 ng to 94 mg, and the dosage may be 50 µL to 100 µL.

The ocular neovascular disease may be any one or more diseases selected from corneal neovascularization, diabetic retinopathy, choroidal neovascularization, and age-related macular degeneration.

The composition of the present disclosure may be prepared in a unit dose form by formulating it using a pharmaceutically acceptable carrier or may be prepared by being contained in a multi-dose container, according to a method that may be easily performed by a person having ordinary skill in the art to which the present disclosure pertains.

The pharmaceutically acceptable carriers are those commonly used in formulations, and include lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil, but are not limited thereto. In addition to the above components, the pharmaceutical composition of the present disclosure may further include a lubricant, a wetting agent, a sweetener, a flavoring agent, an emulsifier, a suspending agent, a preservative, and the like.

In the present disclosure, the content of the additives included in the composition is not particularly limited and may be appropriately adjusted within the content range used in conventional formulations.

The composition of the present disclosure may further include a pharmaceutically acceptable carrier and a diluent for formulation. The pharmaceutically acceptable carriers and diluents include excipients such as starch, sugar, and mannitol, fillers and bulking agents such as calcium phosphate, cellulose derivatives such as carboxymethylcellulose and hydroxypropylcellulose, binders such as gelatin, alginates, and polyvinyl pyrrolidone, lubricants such as talc, calcium stearate, hydrogenated castor oil, and polyethylene glycol, disintegrants such as povidone and crospovidone, and surfactants such as polysorbates, cetyl alcohol, and glycerol, but are not limited thereto. The pharmaceutically acceptable carriers and diluents may be biologically and physiologically compatible with the subject. Examples of diluents include saline, aqueous buffers, solvents, and/or dispersion media, but are not limited thereto.

The term "prevention" as used herein refers to any action of inhibiting or delaying a disease by administering the composition according to the present disclosure. The term "treatment" as used herein refers to any action that alleviates or beneficially alters the symptoms of a disease by administering the composition according to the present disclosure. The term "improvement" as used herein refers to any action that ameliorates the deteriorating condition of a disease by administering or ingesting the composition of the present disclosure to a subject.

### Modes for Carrying Out the Invention

Hereinafter, in order to help understand the present disclosure, embodiments will be given in detail. However, the following embodiments are only intended to illustrate the content of the present disclosure and the scope of the present disclosure is not limited to the following embodiments. These embodiments are provided to more fully explain the present disclosure to those skilled in the art.

### <Preparation Example> Preparation of eye drop compositions

The eye drop composition of the present disclosure includes collagen type I and the PEDF peptide represented by SEQ ID NO: 1, and was prepared under 12 conditions as described below.

Collagen type I was prepared using 4 different collagens from different manufacturers and biological sources. Preparation Example 1, 2, 3, or 5 uses Matrixen^{™}-PSP (HYUNDAI BIOLAND Co., Ltd., Porcine) as collagen type I, Preparation Example 3 or 4 uses TheraCol (Sewon Cellontech Co., Ltd., Porcine), Preparation Example 4 uses VitroCol (ADVANCED BIOMETRIX, Human), and Preparation Example 6, 7, 8, 9, 10, 11, or 12 uses Pepsin Soluble Collagen (COLLAGEN SOLUTIONS, Bovine).

The PEDF peptide represented by SEQ ID NO: 1 was prepared using 4 manufacturing numbers FSC04053, FSC04084, AW19111, or 23-22-0147-01 manufactured by PolyPeptide Laboratories. Preparation Example 1, 2, 3, 4, 5, 6, 7, 8, or 10 uses manufacturing number FSC04053, Preparation Example 9 uses manufacturing number FSC04053, FSC04084, AW19111, or 23-22-0147-01, and Preparation Example 11 or 12 uses manufacturing number 23-22-0147-01.

### Preparation Example 1. Eye drop composition under 1x A5S buffer conditions

An eye drop composition under 1x A5S buffer conditions was prepared in a 2x A5S buffer containing 20 mM sodium acetate, 10% sorbitol, and 0.02% polysorbate 80, and adjusted to pH 5.0±0.1 with acetic acid, such that the final concentration of collagen type I (Matrixen^{™}-PSP, Porcine) was 0.1%, and the final eye drop concentration of the PEDF peptide represented by SEQ ID NO: 1 (manufacturing number FSC04053) was 1 µM, 5 µM, or 25 µM, wherein 10 µL is used for instillation.

The dosage for evaluating efficacy of the prepared eye drop composition was 1.88 µg/kg/day (37.6 ng/eye/day), 9.4 µg/kg/day (188 ng/eye/day), or 47.0 µg/kg/day (940 ng/eye/day) in mice. When the dosage set for mice is converted to a human equivalent dose, it corresponds to a range of 3.13 ng/kg/day (188 ng/eye/day) to 40 µg/kg/day (2.35 mg/eye/day). The PEDF peptide represented by SEQ ID NO: 1 is 3.7632 kDa, and the amount is 37.632 ng/eye/day when 1 µM is instilled at 10 µL.

### Preparation Example 2. Eye drop compositions under 1x A55S buffer conditions

An eye drop composition under 1× A55S buffer conditions was prepared using a 2× A55S buffer containing 20 mM sodium acetate, 10% sorbitol, and 0.02% polysorbate 80, the pH of which was adjusted to 5.5 ± 0.1 using acetic acid, such that the final concentration of porcine-derived collagen type I (Matrixen^{™}-PSP or TheraCol) from different manufacturers was 0.1%, and the final concentration of the PEDF peptide represented by SEQ ID NO: 1 (manufacturing number FSC04053) was 0.2 µM, 1 µM, 5 µM, 25 µM, 50 µM/10 µL, 100 µM, 200 µM, or 400 µM, wherein 10 µL is used for instillation.

The dosage for evaluating efficacy of the prepared eye drop composition was 0.376 µg/kg/day (7.52 ng/eye/day), 1.88 µg/kg/day (37.6 ng/eye/day), 9.4 µg/kg/day (188 ng/eye/day), 47 µg/kg/day (940 ng/eye/day), 94 µg/kg/day (1.88 µg/eye/day), 188 µg/kg/day (3.76 µg/eye/day), 376 µg/kg/day (7.52 µg/eye/day), or 752 µg/kg/day (15 µg/eye/day). When the dosage set for mice is converted to a human equivalent dose, it corresponds to a range of 0.63 ng/kg/day (37.6 ng/eye/day) to 0.63 mg/kg/day (37.6 mg/eye/day).

### Preparation Example 3. Eye drop compositions under 0.1x PBS buffer conditions

An eye drop composition was prepared by diluting 1× PBS (phosphate buffered saline) to 0.1% concentration with distilled water to prepare 0.1× PBS buffer, mixing collagen type I (Matrixen^{™}-PSP, Porcine) such that the final concentration was 0.1%, and mixing the PEDF peptide represented by SEQ ID NO: 1 (manufacturing number FSC04053) such that the final concentration was 1 µM, 5 µM, or 25 µM, wherein 10 µL is used for instillation.

The dosage for evaluating efficacy of the prepared eye drop composition was 1.88 µg/kg/day (37.6 ng/eye/day), 9.4 µg/kg/day (188 ng/eye/day), or 47 µg/kg/day (940 ng/eye/day). When the dosage set for mice is converted to a human equivalent dose, it corresponds to a range of 3.13 ng/kg/day (188 ng/eye/day) to 40 µg/kg/day (2.35 mg/eye/day).

### Preparation Example 4. Eye drop compositions prepared with collagen type I having different manufacturers and biological sources

An eye drop composition was prepared by using 2x A55S buffer to the final concentration of 1x so that the pH of the final eye drop composition was 5.5±0.3, containing 20 mM sodium acetate, 10% sorbitol, and 0.02% polysorbate 80, mixing the PEDF peptide represented by SEQ ID NO: 1 (manufacturing number FSC04053) to the final concentration of 5 µM, and using and mixing TheraCol (Porcine), Pepsin Soluble Collagen (Bovine), or VitroCol (Human) as collagen type I to the final concentration of 0.1%, wherein 10 µL is used for instillation.

For TheraCol, the pH of the final eye drop composition was adjusted to 5.31 using 2x AS buffer adjusted to pH 5.5 with acetic acid, for Pepsin Soluble Collagen, the pH of the final eye drop composition was adjusted to 5.29 using 2x AS buffer adjusted to pH 6.91 with sodium hydroxide, and for VitroCol, the pH of the final eye drop composition was adjusted to 5.30 using 2x AS buffer adjusted to pH 11.59 with sodium hydroxide.

The dosage for evaluating efficacy of the prepared eye drop composition was 9.4 µg/kg/day (188 ng/eye/day). When the dosage set for mice is converted to a human equivalent dose, it corresponds to a range of 15.7 ng/kg/day (940 ng/eye/day) to 7.83 µg/kg/day (470 µg/eye/day).

### Preparation Example 5. Eye drop compositions with two types of collagen type I having different manufacturers and biological sources and three pH conditions

An eye drop composition was prepared by using a 2× AS buffer containing 20 mM sodium acetate, 10% sorbitol, and 0.02% polysorbate 80, adjusted to pH 5.0, 5.5, or 6.0 with acetic acid, such that the final concentration was 1×, using and mixing Pepsin Soluble Collagen (Bovine) or Matrixen^{™}-PSP (Porcine) as collagen type I to the final concentration of 0.1%, and mixing the PEDF peptide represented by SEQ ID NO: 1 (manufacturing number FSC04053) to the final concentration of 5 µM, wherein 10 µL is used for instillation. The final pH of the eye drop composition prepared with Matrixen^{™}-PSP was 4.90, 5.31, or 5.60, and the final pH of the eye drop composition prepared with Pepsin Soluble Collagen was 4.68, 4.96, or 5.10.

The dosage for evaluating efficacy of the prepared eye drop composition was 9.4 µg/kg/day (188 ng/eye/day). When the dosage set for mice is converted to a human equivalent dose, it corresponds to a range of 15.7 ng/kg/day (940 ng/eye/day) to 7.83 µg/kg/day (470 µg/eye/day).

### Preparation Example 6. Eye drop compositions prepared with pepsin soluble collagen under various pH conditions

An eye drop composition was prepared by using a 2× AS buffer containing 20 mM sodium acetate, 10% sorbitol, and 0.02% polysorbate 80, adjusted to pH 4.0, 4.2, 4.5, 4.7, 5.0, or 5.3 with acetic acid, such that the final concentration became 1×, using and mixing collagen type I (Pepsin Soluble Collagen, Bovine) to have the final concentration of 0.1%, and mixing the PEDF peptide represented by SEQ ID NO: 1 (manufacturing number FSC04053) to have the final concentration of 5 µM, wherein 10 µL is used for instillation. The final pH of the eye drop composition is 3.89, 4.04, 4.29, 4.48, 4.65, or 4.85.

The dosage for evaluating efficacy of the prepared eye drop composition was 9.4 µg/kg/day (188 ng/eye/day). When the dosage set for mice is converted to a human equivalent dose, it corresponds to a range of 15.7 ng/kg/day (940 ng/eye/day) to 7.83 µg/kg/day (470 µg/eye/day).

### Preparation Example 7. Eye drop compositions with preservative BAK or PQ1 added

An eye drop composition was prepared by using a 2× AS buffer containing 20 mM sodium acetate, 10% sorbitol, 0.02% polysorbate 80, and as a preservative, 0.01% or 0.02% BAK or 0.002% PQ-1, adjusted to pH 5.3 ± 0.2 with acetic acid, such that the final concentration became 1×, using and mixing collagen type I (Pepsin Soluble Collagen, Bovine) to have the final concentration of 0.1%, and mixing the PEDF peptide represented by SEQ ID NO: 1 (manufacturing number FSC04053) to have the final concentration of 5 µM or 200 µM, wherein 10 µL is used for instillation.

The dosage for evaluating efficacy of the prepared eye drop composition was 9.4 µg/kg/day (188 ng/eye/day) or 376 µg/kg/day (7.52 µg/eye/day). When the dosage set for mice is converted to a human equivalent dose, it corresponds to a range of 15.7 ng/kg/day (940 ng/eye/day) to 0.31 mg/kg/day (18.8 mg/eye/day).

### Preparation Example 8. Eye drop compositions with various peptide concentrations with preservative BAK added

An eye drop composition was prepared by using a 2× A5S buffer containing 20 mM sodium acetate, 10% sorbitol, and 0.02% polysorbate 80, and 0.02% BAK as a preservative, adjusted to pH 5.0 with acetic acid, such that the final concentration became 1×, using and mixing collagen type I (Pepsin Soluble Collagen, Bovine) to have the final concentration of 0.1%, and mixing the PEDF peptide represented by SEQ ID NO: 1 (manufacturing number FSC04053) to have the final concentration of 0.005 µM, 0.05 µM, 0.5 µM, 5 µM, 50 µM, 200 µM, 500 µM, or 1000 µM, wherein 10 µL is used for instillation.

The dosage for evaluating efficacy of the prepared eye drop composition was 9.4 ng/kg/day (0.188 ng/eye/day), 94 ng/kg/day (1.88 ng/eye/day), 940 ng/kg/day (18.8 ng/eye/day), 9.4 µg/kg/day (188 ng/eye/day), 94 µg/kg/day (1.88 µg/eye/day), 376 µg/kg/day (7.52 µg/eye/day), 940 µg/kg/day (18.8 µg/eye/day) or 1.88 mg/kg/day (37.6 µg/eye/day). When the dosage set for mice is converted to a human equivalent dose, it corresponds to a range of 15.7 pg/kg/day (0.94 ng/eye/day) to 1.57 mg/kg/day (94 mg/eye/day).

### Preparation Example 9. Eye drop compositions prepared with peptides of four different manufacturing numbers

An eye drop composition was prepared by using a 2× A5S buffer containing 20 mM sodium acetate, 10% sorbitol, and 0.02% polysorbate 80, and 0.02% BAK as a preservative, adjusted to pH 5.0 with acetic acid, such that the final concentration became 1×, using and mixing collagen type I (Pepsin Soluble Collagen, Bovine) to have the final concentration of 0.1%, and mixing the PEDF peptide represented by SEQ ID NO: 1 to have the final concentration of 5 µM, wherein 10 µL is used for instillation. The PEDF peptides used in the preparation of the eye drop compositions were manufactured under four different manufacturing numbers (manufacturing numbers FSC04053, FSC04084, AW19111, and 23-22-0147-01).

The dosage for evaluating efficacy of the prepared eye drop composition was 9.4 µg/kg/day (188 ng/eye/day). When the dosage set for mice is converted to a human equivalent dose, it corresponds to a range of 15.7 ng/kg/day (940 ng/eye/day) to 7.83 µg/kg/day (470 µg/eye/day).

### Preparation Example 10. Eye drop compositions under 0.025% to 0.2% conditions of collagen type I

An eye drop composition was prepared by using a 2× A5S buffer containing 20 mM sodium acetate, 10% sorbitol, and 0.02% polysorbate 80, adjusted to pH 5.0 ± 0.1 with acetic acid, such that the final concentration became 1×, mixing the PEDF peptide represented by SEQ ID NO: 1 (manufacturing number FSC04053) to have the final concentration of 5 µM, and using and mixing collagen type I (Pepsin Soluble Collagen, Bovine) to have the final concentration of 0.025%, 0.05%, 0.075%, 0.1%, or 0.2%, wherein 10 µL is used for instillation. The pH of the final eye drop composition depending on the concentration of collagen type I (Pepsin Soluble Collagen, Bovine) is 4.89, 4.79, 4.71, 4.63, or 4.35.

The dosage for evaluating efficacy of the prepared eye drop composition was 9.4 µg/kg/day (188 ng/eye/day). When the dosage set for mice is converted to a human equivalent dose, it corresponds to a range of 15.7 ng/kg/day (940 ng/eye/day) to 7.83 µg/kg/day (470 µg/eye/day).

### Preparation Example 11. Eye drop compositions under 0.001% to 0.1% conditions of collagen type I

An eye drop composition was prepared by using a 2× A5S buffer containing 20 mM sodium acetate, 10% sorbitol, and 0.02% polysorbate 80, and 0.02% BAK as a preservative, adjusted to pH 5.0 with acetic acid, such that the final concentration became 1×, mixing the PEDF peptide represented by SEQ ID NO: 1 (manufacturing number 23-22-0147-01) to have the final concentration of 5 µM, and using and mixing collagen type I (Pepsin Soluble Collagen, Bovine) to have the final concentration of 0.001%, 0.005%, 0.01%, 0.05%, or 0.1%, wherein 10 µL is used for instillation. The pH of the final eye drop composition depending on the concentration of collagen type I (Pepsin Soluble Collagen, Bovine) is 5.17, 5.15, 5.12, 4.99, or 4.85.

The dosage for evaluating efficacy of the prepared eye drop composition was 9.4 µg/kg/day (188 ng/eye/day). When the dosage set for mice is converted to a human equivalent dose, it corresponds to a range of 15.7 ng/kg/day (940 ng/eye/day) to 7.83 µg/kg/day (470 µg/eye/day).

### Preparation Example 12. Eye drop compositions for storage stability evaluation

In order to evaluate the storage stability of the final eye drop composition exhibiting CNV inhibitory efficacy, a preparation A was prepared by dissolving the PEDF peptide represented by SEQ ID NO: 1 (manufacturing number 23-22-0147-01) in water for injection to have the final concentration of 800 µM, a preparation B was prepared as a 2× A5S buffer containing 20 mM sodium acetate, 10% sorbitol, 0.02% polysorbate 80, and 0.02% BAK as a preservative, adjusted to pH 5.0 ± 0.3 with acetic acid, and a preparation C was prepared as 6 mg/mL collagen type I (Pepsin Soluble Collagen, Bovine).

To prepare 3 mL of an eye drop composition of 50 µM, collagen type I 0.1%, 188 µL of preparation A, 1500 µL of preparation B, 500 µL of preparation C, and 812 µL of water for injection are mixed.

To prepare 3 mL of an eye drop composition of 200 µM, collagen type I 0.1%, 752 µL of preparation A, 1500 µL of preparation B, 500 µL of preparation C, and 248 µL of water for injection are mixed.

### <Example 1> Evaluation of therapeutic or improvement efficacy of each eye drop composition prepared with collagen type I Matrixen^{™}-PSP in a CNV formation animal model

In order to identify the eye drop composition with the most excellent therapeutic or improvement effect against CNV among the eye drop compositions prepared according to each preparation example of the present disclosure, a CNV formation animal model was established, and the improvement effect on neovascular lesions according to administration of each eye drop composition was evaluated. The animal model used in the experiment was a 5-week-old C57BL/6 mouse, purchased from Nara Biotech, and used in the present experiment at 6 to 8 weeks of age after a breeding adaptation period.

The mouse pupils were dilated by instilling 5 mg/mL tropicamide (Santen Pharmaceutical Co. Ltd., Kita-ku, Osaka, Japan) into the eyes, and the mouse was anesthetized by intraperitoneal injection of 100 mg/kg ketamine (Huons, SEONGNAM-SI, GYEONGGI-DO, Korea) and 10 mg/kg xylazine (BAYER, Leverkusen, Germany). After anesthesia, the mouse was fixed on a mouse fixture, and a 210 mW laser was irradiated for 0.1 s using a slit lamp microscope laser system to create a total of four to five lesions of 100 µm in size in the 3, 6, 9, and 12 o'clock directions on the mouse retina.

In the mouse model in which a lesion was created in the retina, each eye drop composition in the preparation example was instilled with 10 µL into the right eye once a day for 14 days, such that the PEDF peptide was contained at a dosage of 1.88 µg/kg/day (37.6 ng/eye/day), 9.4 µg/kg/day (188 ng/eye/day), or 47 µg/kg/day (940 ng/eye/day).

In the control group, 4 µg/2 µl of Eylea (Bayer AG) was injected through intravitreal injection (IVT) using a 35 Ultra Micropump and Microcontroller system. After laser irradiation, mice receiving each eye drop for 14 days were anesthetized with ketamine (100 mg/kg) and xylazine (10 mg/kg), and 100 µL of 25 mg/mL FITC-dextran (Sigma-Aldrich, St. Louis, MO, USA) was injected retro-orbitally to stain the retinal blood vessels for 30 minutes. The eyes were then enucleated, fixed in 10% formalin for 1 hour at room temperature, and the muscles, cornea, iris, lens, and vitreous around the fixed eyes were carefully removed under a microscope without damaging the retina. After making four incisions in the 3, 6, 9, and 12 o'clock directions, coverslips were applied, and lesions were photographed using a fluorescence microscope, and the lesion areas were measured and compared with the control group.

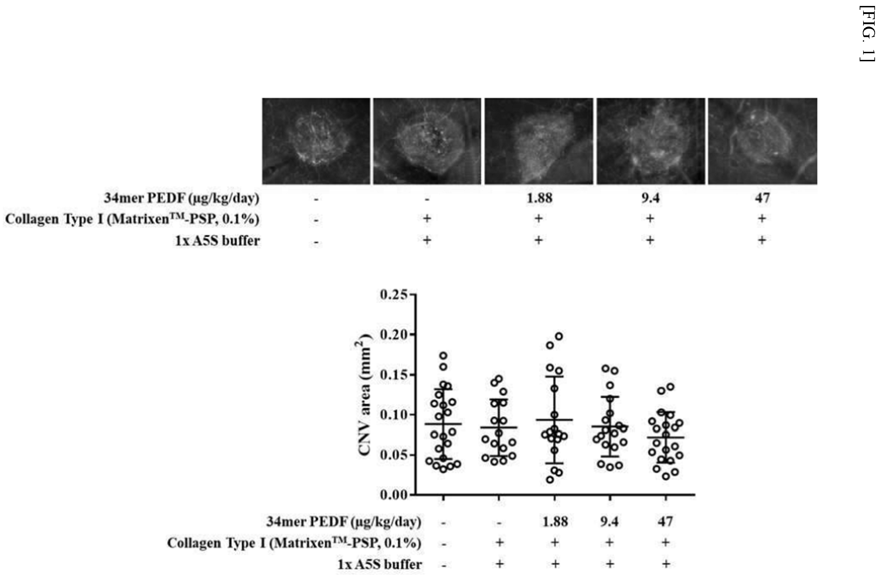

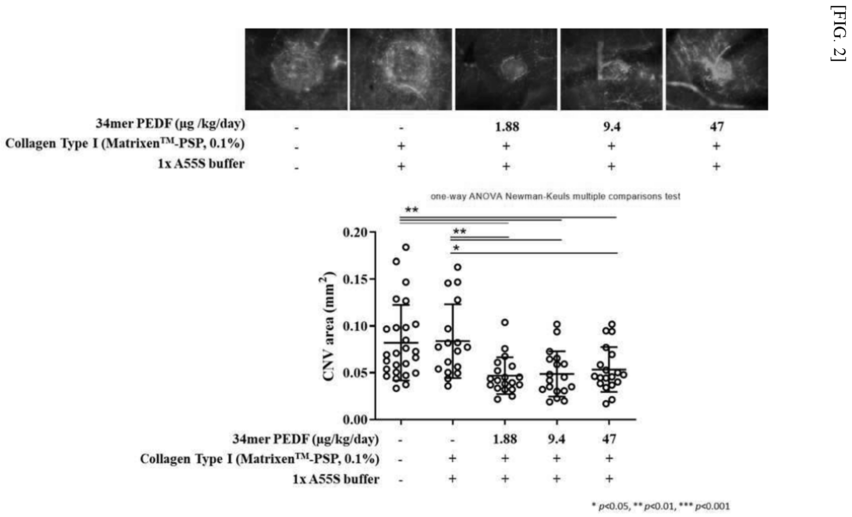

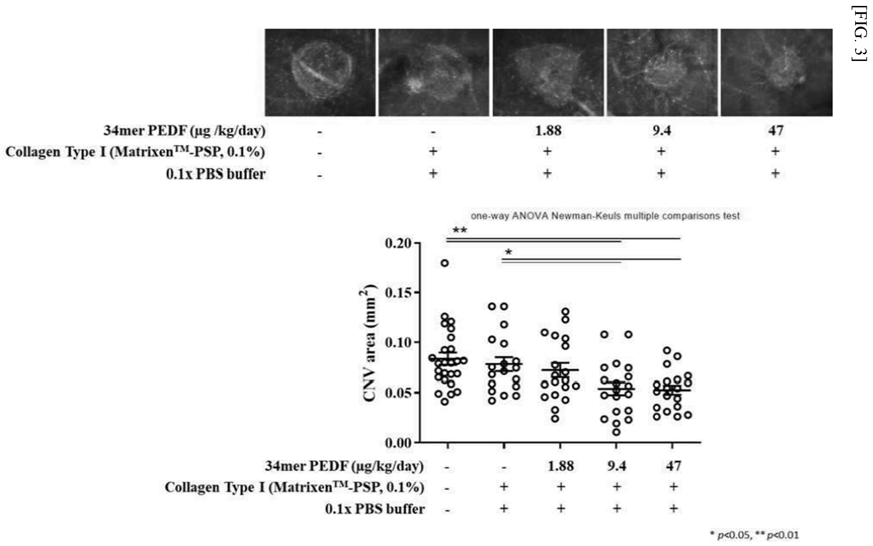

In the case of mice administered with eye drop compositions under 1x A5S buffer conditions prepared according to Preparation Example 1, the effect of inhibiting CNV was not significantly observed compared to the control group under all conditions as shown in FIG. 1. On the other hand, in the case of mice administered with eye drop compositions under 1x A55S buffer conditions prepared according to Preparation Example 2, as shown in FIG. 2, when the content of PEDF peptide in the eye drop composition under 1x A55S buffer conditions was 1.88 µg/kg/day (37.6 ng/eye/day), 9.4 µg/kg/day (188 ng/eye/day), or 47 µg/kg/day (940 ng/eye/day), a significant reduction effect of the area of CNV by 57.1±23.9%, 59.4±29.5%, or 65.1±29.1% was observed. In the case of mice administered with eye drop compositions under 0.1x PBS buffer conditions prepared according to Preparation Example 3, as shown in FIG. 3, when the content of PEDF peptide in the eye drop composition under 0.1x PBS buffer conditions was 9.4 µg/kg/day (188 ng/eye/day) and 47 µg/kg/day (940 ng/eye/day), the area of CNV was significantly reduced by 64.4±32.9% or 62.9±23.4%, but when the content was 1.88 µg/kg/day (37.6 ng/eye/day), the area of CNV was 86.5±37.0%, showing a low effect of inhibiting CNV.

In summary of the above results, the effect of inhibiting CNV was found to be the best in the eye drop composition prepared according to Preparation Example 2 under the condition of 1x A55S buffer, and the effect of inhibiting CNV was also found to be excellent under the condition of the eye drop composition containing a low concentration of PEDF peptide of 1.88 µg/kg/day (37.6 ng/eye/day).

### <Example 2> Evaluation of therapeutic efficacy concentration range in the CNV formation animal model

Referring to the results of Example 1, the concentration range of the PEDF peptide exhibiting an inhibitory effect on CNV was determined using the eye drop composition prepared under 1x A55S buffer conditions according to Preparation Example 2, which showed the highest efficacy. The laser-induced CNV formation model was constructed and lesion analysis was performed using the same methods as in Example 1. The eye drop compositions under 1x A55S buffer conditions prepared according to Preparation Example 2 were instilled with 10 µL to the right eye once a day for 14 days, such that the PEDF peptide was contained at a dosage of 0.376 µg/kg/day (7.52 ng/eye/day), 1.88 µg/kg/day (37.6 ng/eye/day), or 9.4 µg/kg/day (188 ng/eye/day).

For comparison of efficacy, mice that were instilled the eye drop composition under 0.1x PBS buffer conditions prepared according to Preparation Example 3 once a day for 14 days were used as a control group, and the eye drop composition of the control group was instilled with 10 µL once a day to the right eye for 14 days, such that the PEDF peptide was contained in the dosage at 9.4 µg/kg/day (188 ng/eye/day).

On the 14th day after laser irradiation, the mice in each experimental group were anesthetized using ketamine (100 mg/kg) and xylazine (10 mg/kg), and 50 µL of 10% Fluorescite (Alcon, Fort Worth, TX, USA) was injected retro-orbitally to stain the retinal blood vessels for 5 minutes. Thereafter, the eyes were enucleated, flat-mounted in the same manner as in Example 1, and the lesion area was measured after photographing the lesion and compared with the control group.

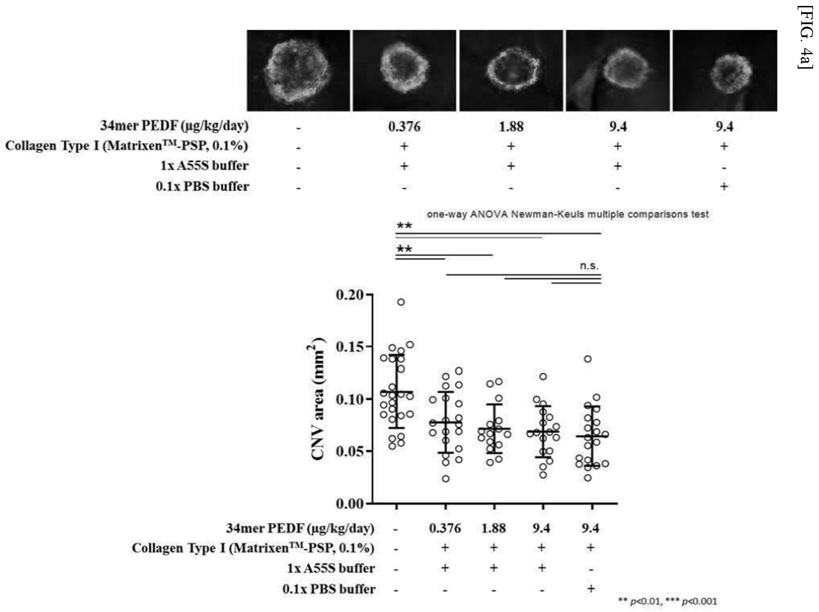

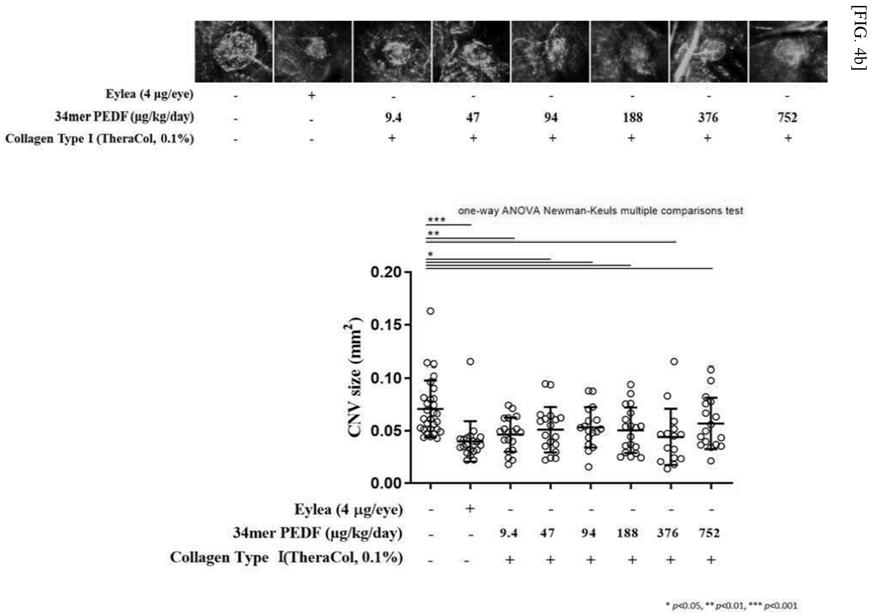

In the case of mice administered with eye drop compositions under 1x A55S buffer conditions prepared according to Preparation Example 2, as shown in FIG. 4A, when the content of PEDF peptide was 0.376 µg/kg/day (7.52 ng/eye/day), 1.88 µg/kg/day (37.6 ng/eye/day), or 9.4 µg/kg/day (188 ng/eye/day), there was an effect of significantly reducing the area of CNV by 72.6±26.6%, 66.9±21.1%, or 64.2±22.3%. Additionally, when the content of PEDF peptide was increased to 752 µg/kg/day (15 µg/eye/day), as shown in FIG. 4B, when the eye drop composition under 1x A55S buffer conditions prepared according to Preparation Example 2 was administered, the area of CNV was significantly reduced by 65.62±23%, 72.19±30.49%, 75.36±27.08%, 71.42±30.48%, or 62.28±37.97% when PEDF peptide (manufacturing number FSC04053) was administered at 9.4 µg/kg/day (188 ng/eye/day), 47 µg/kg/day (940 ng/eye/day), 94 µg/kg/day (1.88 µg/eye/day), 188 µg/kg/day (3.76 µg/eye/day), or 376 µg/kg/day (7.52 µg/eye/day). On the other hand, when the PEDF peptide content was 752 µg/kg/day (15 µg/eye/day), the area of CNV was 80.64±34.59%, showing low inhibitory effect.

In summary of the above results and the results of Example 1, the content of PEDF peptide (manufacturing number FSC04053) in the eye drop composition under 1x A55S buffer conditions prepared according to Preparation Example 4 showed an excellent CNV inhibitory effect when the range was 0.376 µg/kg/day (7.52 ng/eye/day) to 376 µg/kg/day (7.52 µg /eye/day) with respect to the total eye drop composition weight.

### <Example 3> Evaluation of therapeutic or improvement efficacy range according to the type of collagen type 1 in the CNV formation animal model

In order to identify the eye drop composition with the most excellent therapeutic or improvement effect against CNV among the eye drop compositions prepared according to each preparation example of the present disclosure, the CNV inhibitory efficacy according to the type of collagen type I was examined using the eye drop composition under 1x A55S buffer conditions. The method for creating a laser-induced CNV formation model and analyzing lesions was performed using the same method as in Example 1. Eye drop compositions under 1x A55S buffer conditions prepared with collagen type I from different manufacturers according to Preparation Example 4 or 5 were instilled with 10 µL to the right eye once a day for 14 days, such that the PEDF peptide (manufacturing number FSC04053) was contained in the dosage at 9.4 µg/kg/day (188 ng/eye/day).

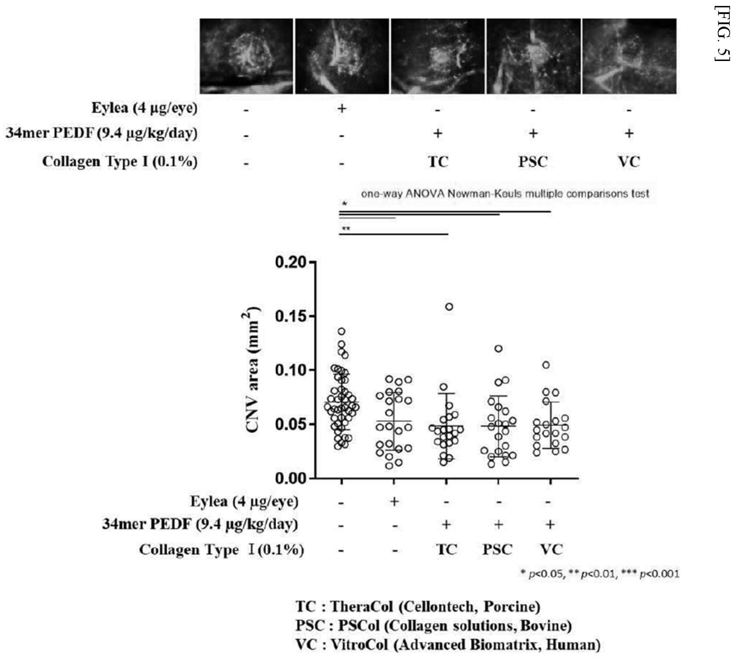

In the case of mice administered with eye drop compositions prepared with three types of collagen type I prepared according to Preparation Example 4, as shown in FIG. 5, when the collagen type I was TheraCol (Porcine), Pepsin Soluble Collagen (Bovine), or VitroCol (Human), the area of CNV was significantly reduced by 68.3±42.4%, 68.1±39.7%, or 69.7±30.3%.

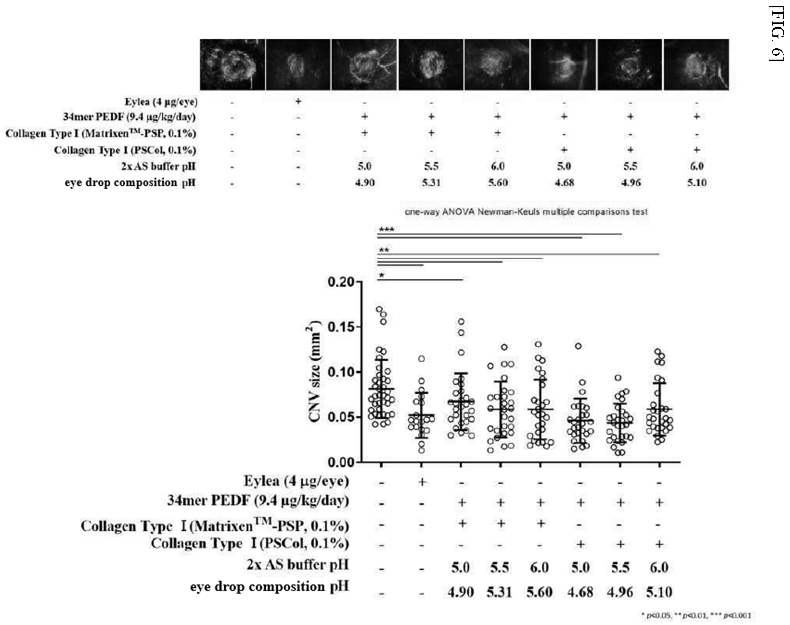

In the case of mice administered with eye drop compositions with two types of collagen type I and three pH conditions prepared according to Preparation Example 5, as shown in FIG. 6, in the eye drop composition prepared using Pepsin Soluble Collagen (Bovine) as collagen type I, the area of CNV was significantly reduced by 56.4±30.1%, 53.5±26.2%, or 72.0±35.9% when the pH of the 2x AS buffer was 5.0, 5.5, or 6.0. On the other hand, in the eye drop composition prepared using collagen type I (Matrixen^{™}-PSP, Porcine), when the pH of the 2x AS buffer was 5.0, the area of CNV was 82.2±38.8%, showing a low inhibitory effect, but when the pH of the 2x AS buffer was 5.5 or 6.0, the effect of significantly reducing the area of CNV by 71.9±37.9% or 71.9±40.7% was shown.

In summary of the above results, it was confirmed that the eye drop compositions prepared with TheraCol (Porcine), VitroCol (Human), or Pepsin Soluble Collagen (Bovine) showed almost the same inhibitory efficacy on CNV at 9.4 µg/kg/day (188 ng/eye/day) of PEDF peptide (manufacturing number FSC04053). Additionally, when a comparative experiment on the inhibitory efficacy on CNV according to pH was conducted, Matrixen^{™}-PSP (Porcine) did not show any inhibitory efficacy on CNV at pH 5.0, but only at pH 5.5 and 6.0, whereas Pepsin Soluble Collagen (Bovine) showed inhibitory efficacy on CNV at all pHs 5.0, 5.5, and 6.0.

### <Example 4> Evaluation of therapeutic or improvement efficacy of eye drops prepared with PEDF peptide (manufacturing number FSC04053) in the CNV formation animal model

Referring to the results of Example 3, in order to identify the eye drop composition with the most excellent therapeutic or improvement effect against CNV in the eye drop compositions prepared with collagen type I (Pepsin Soluble Collagen, Bovine), the inhibitory efficacy on CNV was evaluated according to the concentration of PEDF peptide (manufacturing number FSC04053), the concentration of collagen type I, the pH of AS buffer, or the presence or absence of preservatives.

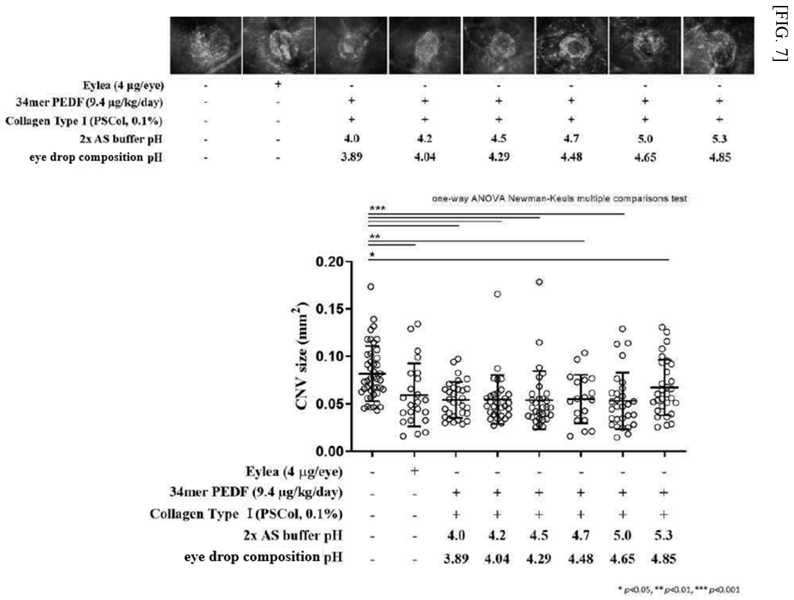

In the case of mice administered with eye drop compositions of various pH AS buffer conditions prepared with Pepsin Soluble Collagen (Bovine) according to Preparation Example 6, as shown in FIG. 7, when the pH of the 2x AS buffer was 4.0, 4.2, 4.5, 4.7, 5.0, or 5.3, the effect of significantly reducing the area of CNV by 66.3±23.1%, 66.9±31.4%, 66.0±37.2%, 67.4±31.2%, or 65.1±36.1% was shown, but when the pH was 5.3, the area of CNV was 82.0±35.6%, showing a low CNV inhibitory effect.

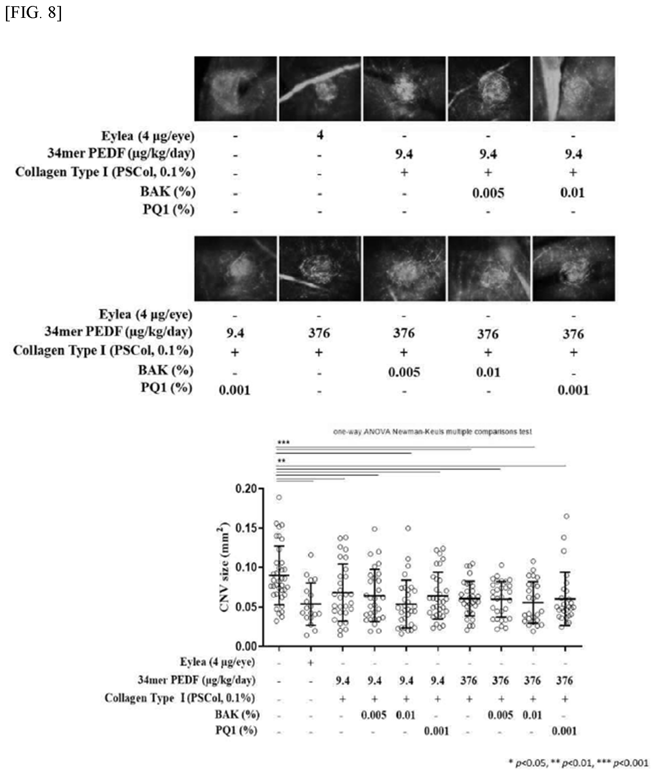

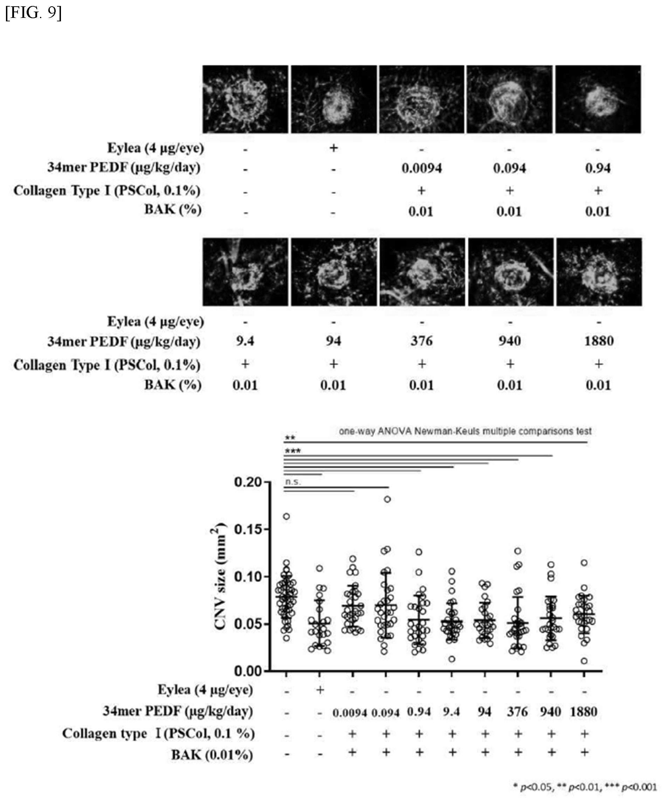

In the case of mice administered with eye drop compositions with a preservative added under 1x A55S buffer conditions according to Preparation Example 7, as shown in FIG. 8, when the content of PEDF peptide in the eye drop composition under 1x A55S buffer conditions was 9.4 µg/kg/day (188 ng/eye/day) or 376 µg/kg/day (7.52 µg/eye/day) and 0.005% of the preservative BAK was added, the effect of significantly reducing the area of CNV by 71.4±36.6% or 59.9±33.6% was shown, and when 0.01% of the preservative BAK was added, the area of CNV was significantly reduced by 66.5±25.1% or 62.1±29.2%. Even when 0.01% of the preservative PQ1 was added, the area of CNV was significantly reduced by 71.2±32.8% or 67.0±37.1%. Referring to the results shown in FIG. 9, the effective concentration range of PEDF peptide in the eye drop composition prepared using 0.01% BAK as a preservative was evaluated.

In the case of mice administered with eye drop compositions with various concentrations of PEDF peptide to which the preservative BAK was added according to Preparation Example 8, as shown in FIG. 9, when the content of PEDF peptide in the eye drop composition under 1x A5S buffer conditions to which the preservative BAK 0.01% was added was 9.4 ng/kg/day (0.188 ng/eye/day) or 94 ng/kg/day (1.88 ng/eye/day), the area of CNV was 87.9±27.5% or 88.8±43.6%, showing a low CNV inhibitory effect. When the content of PEDF peptide was 940 ng/kg/day (18.8 ng/eye/day), 9.4 µg/kg/day (188 ng/eye/day), 94 µg/kg/day (1.88 µg/eye/day), 376 µg/kg/day (7.52 µg/eye/day), 940 µg/kg/day (18.8 µg/eye/day), or 1.88 mg/kg/day (37.6 µg/eye/day), the area of CNV was significantly reduced by 69.1±32.8%, 66.9±24.4%, 68.4±23.6%, 65.3±34.5%, 71.1±29.4%, or 76.4±25.2%, and therefore, when the content of PEDF peptide was 940 ng/kg/day (18.8 ng/eye/day) to 1.88 mg/kg/day (37.6 µg/eye/day), CNV was inhibited, and in particular, when the content was 940 ng/kg/day (18.8 ng/eye/day) to 376 µg/kg/day (7.52 µg/eye/day), the effect of inhibiting CNV was the best.

In summary of the above results, the effect of inhibiting CNV was shown in the condition of the eye drop composition containing 0.01% of BAK as a preservative in 1x A5S buffer, 0.1% of collagen type I (Pepsin Soluble Collagen, Bovine), and 940 ng/kg/day (18.8 ng/eye/day) to 1.88 mg/kg/day (37.6 µg/eye/day) of PEDF peptide (Manufacturing No. FSC04053) according to Preparation Example 8.

### <Example 5> Evaluation of therapeutic or improvement efficacy range of eye drops prepared with PEDF peptides of different manufacturing numbers in the CNV formation animal model

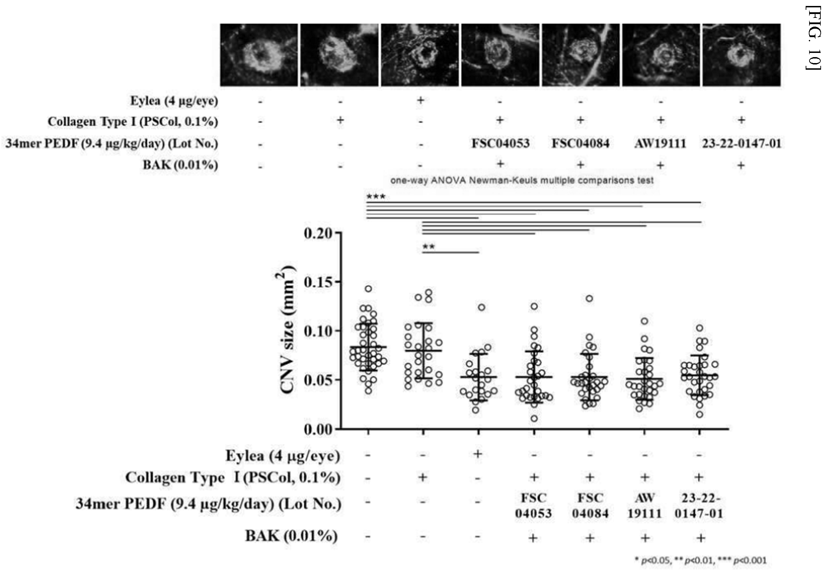

In the case of mice administered with eye drop compositions prepared with PEDF peptides of four manufacturing numbers manufactured by PolyPeptide Laboratories according to Preparation Example 9, as shown in FIG. 10, when the PEDF peptide content was 9.4 µg/kg/day (188 ng/eye/day) and the manufacturing numbers of the peptides were FSC04053, FSC04084, AW19111, or 23-22-0147-01, the effect of significantly reducing the area of CNV by 63.5±31.3%, 63.4±28.4%, 61.2±25.5%, or 65.5±24.3% was shown. In summary of the above results, the effect of inhibiting CNV was excellent regardless of the manufacturing number when the content of PEDF peptide was 9.4 µg/kg/day (188 ng/eye/day).

### <Example 6> Evaluation of the effective concentration range of collagen type 1 in eye drop compositions showing therapeutic or improvement efficacy in the CNV formation animal model

Referring to the results of Examples 4 and 5, an evaluation was conducted to determine the effective concentration range showing the CNV inhibitory efficacy of collagen type I (Pepsin Soluble Collagen, Bovine) among eye drop compositions prepared using the composition having the PEDF peptide content of 9.4 µg/kg/day (188 ng/eye/day) under 1x A5S buffer conditions.

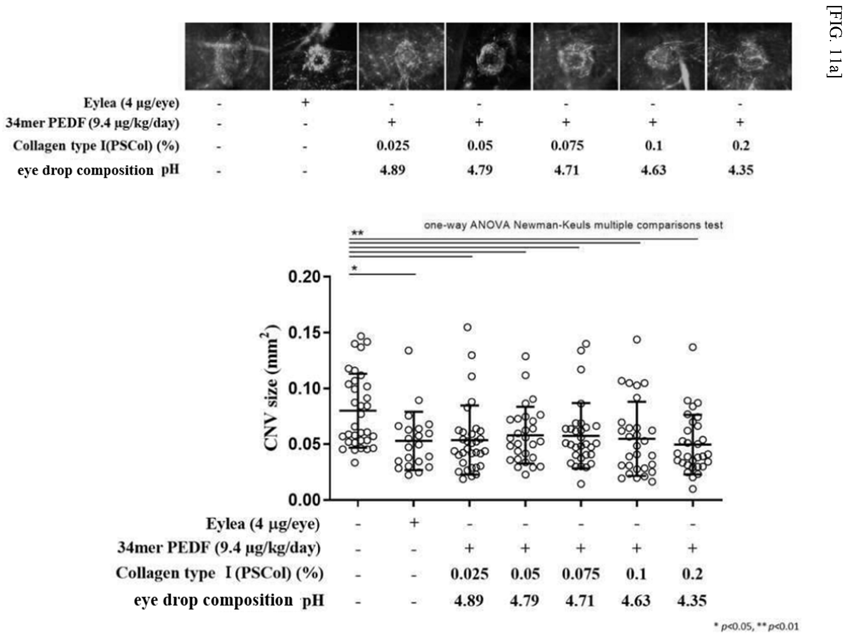

When the eye drop compositions of 0.025% to 0.2% conditions of collagen type I under 1x A5S buffer conditions prepared according to Preparation Example 10 were administered, as shown in FIG. 11A, when the content of PEDF peptide (manufacturing number FSC04053) was 9.4 µg/kg/day (188 ng/eye/day) and the content of collagen type I (Pepsin Soluble Collagen, Bovine) was 0.025%, 0.05%, 0.075%, 0.1%, or 0.2%, the area of CNV was significantly reduced by 67.1±38.7%, 72.5±31.8%, 71.9±36.5%, 68.5±41.4%, or 62.1±33.4%. To determine the minimum concentration of collagen type I (Pepsin Soluble Collagen, Bovine) that significantly reduces CNV, additional experiments were conducted including lower concentrations of 0.001%, 0.005%, or 0.01% than 0.025%.

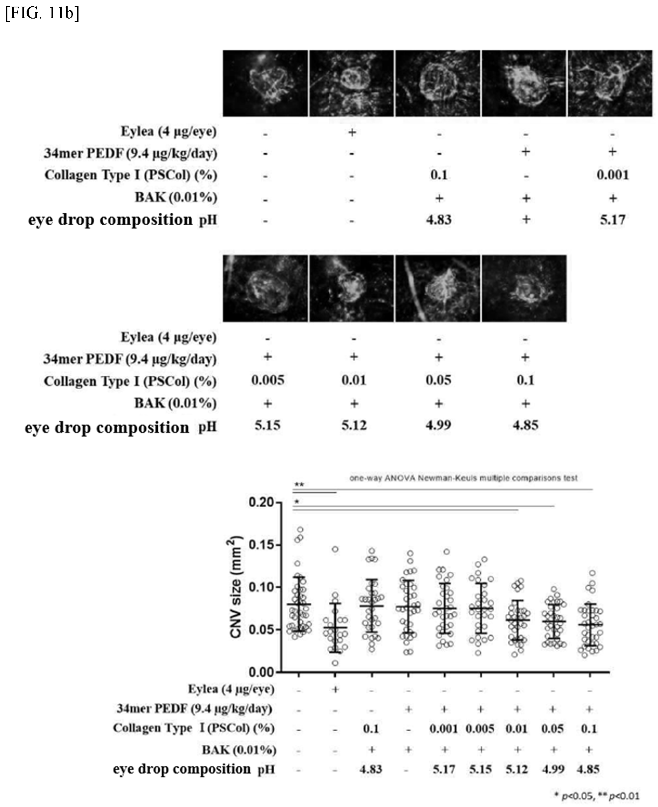

In the case of mice administered with eye drop compositions under 1x A5S buffer conditions having the content of PEDF peptide (manufacturing number 23-22-0147-01) of 9.4 µg/kg/day (188 ng/eye/day) of and the content of collagen type I (Pepsin Soluble Collagen, Bovine) of 0.001% or 0.005% prepared according to Preparation Example 11, as shown in FIG. 11B, the area of CNV was 93.15±36.6% or 93.84±36.7%, showing a low CNV inhibitory effect. On the other hand, when the content of collagen type I (Pepsin Soluble Collagen, Bovine) was 0.01%, 0.05%, or 0.1%, the area of CNV was significantly reduced by 76.53±28.9%, 74.77±24.9%, or 69.91±30.4%.

In summary of the above results, in the eye drop compositions under 1x A5S buffer conditions prepared according to Preparation Example 11, when the content of PEDF peptide was 9.4 µg/kg/day (188 ng/eye/day) based on the total weight of the eye drop composition, and the content of collagen type I (Pepsin Soluble Collagen, Bovine) was 0.01% to 0.2% based on the total weight of the eye drop composition, an excellent CNV inhibitory effect was shown.

### <Example 7> Storage stability evaluation of the final eye drop composition

The final eye drop composition of the present disclosure, prepared according to Preparation Example 12, was stored for 4 weeks under refrigerated conditions (5±3°C) or room temperature conditions (25±2°C), depending on the experimental purpose, and the storage stability was evaluated by visual inspection, pH measurement, and reverse phase ultra-high performance liquid chromatography (RP-UPLC) analysis immediately after preparation, and at weeks 1, 2, 3, and 4, respectively. The appearance of the final eye drop composition prepared according to the present disclosure was analyzed by comparing with a freshly prepared reference solution (ultra-pure water R) through visual inspection, the pH was measured, and the content and purity of the preservative BAK and the PEDF peptide contained in the eye drop composition were evaluated through RP-UPLC analysis using a Shimadzu Nexera ultra-high performance liquid chromatography (UPLC) instrument. For the preservative BAK, the stationary phase of UPLC was Luna 5 µm CN 100 Å, LC column (5 µm / 100 Å 250 X 4.6 mm), and the mobile phase was acetonitrile (ACN) solution containing 0.45% (v/v) 75 mM sodium acetate, pH 5.0 (sodium acetate solution). The analysis was performed under the following conditions: the mobile phase flow rate was 1 mL/min, the sample injection volume was 20 µL, and the ultraviolet spectrophotometer wavelength was 254 nm ± 2 nm. The temperature of the column was controlled at 35°C during the analysis, and the analysis time was 35 minutes.

For PEDF peptides, Waters Acquity Peptide CSH C18* (1.7 µm / 130 Å 100 X 2.1 mm) was used as the stationary phase of UPLC, and Solution A, distilled water containing 0.1% (v/v) trifluoroacetic acid (TFA), and Solution B, acetonitrile (ACN) containing 0.1% (v/v) trifluoroacetic acid (TFA) were used as the mobile phase. The analysis was performed under the following conditions: the mobile phase flow rate was 0.4 mL/min, the sample injection volume was 6 µL, and the ultraviolet spectrophotometer wavelength was 210 nm ± 4 nm. During the analysis, the temperature of the column was controlled at 47°C, and the mixing conditions of the mobile phase solutions according to time were set such that the mixing volume ratio of solution A and solution B was 72:28 for 50 minutes, 62:38 from 51 to 55 minutes, 0:100 from 56 to 59 minutes, and 72:28 from 60 to 75 minutes.

As a result of storing the final eye drop compositions of the present disclosure prepared according to Preparation Example 12 for 4 weeks under refrigerated conditions (5±3°C) and room temperature conditions (25±2°C), as shown in FIG. 12, the visual inspection and pH measurement results showed no changes in transparency, particles, color, or pH, and the RP-UPLC analysis results confirmed that the peaks corresponding to the preservative BAK and the PEDF peptide were observed at the same positions. In summary, the results of analyzing the storage stability according to the storage period of the final eye drop compositions of the present disclosure prepared according to the preparation examples under refrigerated conditions (5±3°C) or room temperature conditions (25±2°C) are as shown in Table 1 below. The results in Table 1 below include errors due to sample storage, and the corresponding error range is sufficient to evaluate the storage safety of the eye drops according to the storage period. The acceptable standard for storage safety was set to the preservative BAK content in the range of 0.01±0.005%, and the decrease in the content of the active ingredient, the peptide, was limited to about 10%, and if the content of the active ingredient is decreased by more than 10%, it means that the composition is not stable.

In summary of the above results, it may be confirmed that the final eye drop compositions of the present disclosure prepared according to Preparation Example 12 may be stably stored for 4 weeks under refrigerated conditions (5±3°C) and room temperature conditions (25±2°C).

**[Table 1]**

| Analysis items | | | Acceptan ce criteria | Test period | | | | |
|---|---|---|---|---|---|---|---|---|
| | | | | T0 | T1W | T2W | T3W | T4W |
| | Appearan ce | Color | Colorless to yellow liquid | Colorless to yellow liquid | Colorless to yellow liquid | Colorless to yellow liquid | Colorless to yellow liquid | Colorless to yellow liquid |
| | | Brightness | Transpar ent to milky white | Transpar ent to milky white | Transpar ent to milky white | Transpar ent to milky white | Transpar ent to milky white | Transpar ent to milky white |
| | | Visible particles | Substanti ally particle-free solution | Substanti ally particle-free solution | Substanti ally particle-free solution | Substanti ally particle-free solution | Substanti ally particle-free solution | Substanti ally particle-free solution |
| | pH | | 5.0 ± 0.3 | 4.8 | 4.8 | 4.8 | 4.8 | 4.9 |
| Low dose | RP-UHPLC | Identity | Compara ble to reference standard | Compara ble to reference standard | Compara ble to reference standard | Compara ble to reference standard | Compara ble to reference standard | Compara ble to reference standard |
| | | PEDF peptide concentrati on (uM) | 50.0 ± 5.0 µM | 50.5 | 50.5 | 49.9 | 49.3 | 51.7 |
| | | PEDF peptide purity (%) | ≥ 90% | 98 | 97 | 94 | 94 | 94 |
| | | Total impurities (%) | < 10% | 2 | 3 | 6 | 6 | 6 |
| | BAK concentration (%) | | 0.01 ± 0.005% | 0.008 | 0.008 | 0.009 | 0.008 | 0.008 |
| High dose | Appearan ce | Color | Colorless to yellow liquid | Colorless to yellow liquid | Colorless to yellow liquid | Colorless to yellow liquid | Colorless to yellow liquid | Colorless to yellow liquid |
| | | Brightness | Transpar ent to milky white | Transpar ent to milky white | Transpar ent to milky white | Transpar ent to milky white | Transpar ent to milky white | Transpar ent to milky white |
| | | Visible particles | Substanti ally | Substanti ally | Substanti ally | Substanti ally | Substanti ally | Substanti ally |
| | | | particle-free solution | particle-free solution | particle-free solution | particle-free solution | particle-free solution | particle-free solution |
| | pH | | 5.0 ± 0.3 | 4.8 | 4.8 | 4.8 | 4.8 | 4.9 |
| | RP-UHPLC | Identity | Compara ble to reference standard | Compara ble to reference standard | Compara ble to reference standard | Compara ble to reference standard | Compara ble to reference standard | Compara ble to reference standard |
| | | PEDF peptide concentrati on (uM) | 200.0 ± 20.0 µM | 195.9 | 199.7 | 194.5 | 192.5 | 191.5 |
| | | PEDF peptide purity (%) | ≥ 90% | 99 | 98 | 95 | 95 | 96 |
| | | Total impurities (%) | < 10% | 1 | 2 | 5 | 5 | 4 |
| | BAK concentration (%) | | 0.01 ± 0.005% | 0.008 | 0.008 | 0.009 | 0.009 | 0.008 |

While specific aspects of the present disclosure have been described in detail above, it should be apparent to those skilled in the art that these specific descriptions merely represent preferred embodiments and are not intended to limit the scope of the present disclosure. In other words, the substantial scope of the present disclosure is defined by the appended claims and their equivalents.

## Claims

1. An eye drop composition for preventing or treating an ocular neovascular disease, comprising collagen type I and a pigment epithelium-derived factor (PEDF) peptide.

2. The eye drop composition of claim 1, wherein the PEDF peptide consists of the amino acid represented by SEQ ID NO: 1.

3. The eye drop composition of claim 1, wherein the eye drop composition comprises the PEDF peptide in a concentration range of 37.6 ng/50 µL to 94 mg/50 µL.

4. The eye drop composition of claim 1, wherein the eye drop composition comprises sodium acetate in a range of 9.9 mM to 10.1 mM.

5. The eye drop composition of claim 1, wherein the eye drop composition comprises sorbitol in a range of 4.9 % (w/v) to 5.1 % (w/v), polysorbate 80 in a range of 0.009 % (w/v) to 0.011 % (w/v), and benzalkonium chloride (BAK) in a range of 0.005 % (w/v) to 0.015 % (w/v).

6. The eye drop composition of claim 1, wherein the eye drop composition has a pH of 5.0 ± 0.3.

7. The eye drop composition of claim 1, wherein the eye drop composition is storable for 4 weeks at a temperature ranging from 1°C to 30°C.

8. The eye drop composition of claim 1, wherein the eye drop composition is instilled into an eye once a day with the PEDF peptide at a dosage ranging from 0.94 ng/eye/day to 94 mg/eye/day.

9. The eye drop composition of claim 8, wherein the dosage is between 50 µL and 100 µL.

10. The eye drop composition of claim 1, wherein the ocular neovascular disease is any one or more diseases selected from corneal neovascularization, diabetic retinopathy, choroidal neovascularization, and age-related macular degeneration.
